# EUROPÄISCHE PATENTSCHRIFT

(11) **EP 1 107 862 B1**
(45) Veröffentlichungstag und Bekanntmachung des Hinweises auf die Patenterteilung: **11.12.2002**
(21) Anmeldenummer: 99945997.7
(22) Anmeldetag: 14.08.1999
(51) Int. Cl.: B30B 11/08, B30B 11/34, B30B 15/06

(54) **HERSTELLUNG MEHRPHASIGER FORMKÖRPER**
PRODUCTION OF MULTIPHASE MOULDED PIECES
PRODUCTION DE CORPS MOULES A PLUSIEURS PHASES

(30) Priorität: 24.08.1998 DE 19838396
(43) Veröffentlichungstag der Anmeldung: 20.06.2001
(73) Patentinhaber: Henkel Kommanditgesellschaft auf Aktien, 40589 Düsseldorf (DE)
(72) Erfinder: BEAUJEAN, Hans-Josef, D-41539 Dormagen (DE); HOLDERBAUM, Thomas, D-40789 Monheim (DE); HÄRER, Jürgen, D-40593 Düsseldorf (DE); NITSCH, Christian, D-40591 Düsseldorf (DE)
(86) Internationale Anmeldenummer: EP9906076
(87) Internationale Veröffentlichungsnummer: WO00010800

(56) Entgegenhaltungen:
- EP-A- 0 307 209
- DE-A- 2 834 226
- FR-A- 1 072 611
- GB-A- 1 034 713
- US-A- 3 840 631
- US-A- 4 057 381
- US-A- 4 419 413
- US-A- 5 158 728

## Beschreibung

Die vorliegende Erfindung betrifft eine Vorrichtung zum Pressen mehrphasiger Formkörper auf einer Rundläuferpresse und ein Herstellverfahren für mehrphasige Formkörper unter Einsatz dieser Vorrichtung. Das erfindungsgemäße Herstellverfahren eignet sich insbesondere für die Herstellung von Wasch- und Reinigungsmittelformkörpern.

Rundlaufpressen zur Herstellung von Tabletten aus den verschiedensten Stoffen und für die unterschiedlichsten Anwendungsgebiete sind aus dem Stand der Technik bekannt. Bei diesen Pressen weist eine um eine (zumeist vertikal stehende) Achse angetriebene Matrizenscheibe auf einem Kreis angeordnete Bohrungen (Matrizen) auf, den synchron mit der Scheibe umlaufende Preßstempelpaare zugeordnet sind. Die Preßstempel werden dabei durch Steuerkurven und Druckrollen abgesenkt bzw. angehoben, so daß die in die Matrize eingefüllte Mischung verdichtet und ausgestoßen wird. Während der Befüllung der Matrizenbohrungen mittel einer geeigneten Füllvorrichtung (Füllschuh) wird der Boden der Matrize durch den Unterstempel gebildet, wobei das Matrizenvolumen und damit die Dosierung der zu tablettierenden Mischung von der Höhe des Unterstempels in der Matrizenbohrung abhängt. Nach dem Einfüllen wird das Vorgemisch durch Absenken des Oberstempels mittels einer Druckrolle bzw, die Aufeinanderzubewegung gegenüberliegender Preßstempel auf eine gewünschte Höhe verdichtet, wobei moderne Preßstationen eine Vordruck- und eine Hauptdruckstation (also mehrere Druckrollen pro Füllschuh) aufweisen. Im Anschluß an die Verdichtung werden Ober- und Unterstempel angehoben, wodurch die Tablette an einer bestimmten Stelle der Maschine aus der Matzrize austritt und durch geeignete Vorrichtungen (Abstreifer) von der Matrizenscheibe entfernt und einem Ablaufkanal zugeführt werden kann. Bei diesem Vorgang hebt sich der Oberstempel im allgemeinen stärker und schneller an als der Unterstempel.

Mit Hilfe der bekannten Rundläuferpressen ist es nicht nur möglich, durchgehend homogen zusammengesetzte ("einphasige") Tabletten herzustellen; vielmehr sind durch Einbau mehrerer Füllschuhe und Druckrollen bzw. Auswurfschienen auch mehrschichtige Tabletten herstellbar. Dabei wird die zuerst eingefüllte Schicht nur leicht vorverpreßt, und die Endverpressung erfolgt nach Befüllen der Matrize mit dem Vorgemisch für die letzte Schicht, um den Zusammenhalt der einzelnen Schichten zu verbessern. Auch die Herstellung anderer geometrischer Phasenaufteilungen wir beispielsweise Manteltabletten oder Ring/Kerntabletten (in der Pharmazie üblicherweise als Punkttabletten oder Bull-eye-Tabletten bezeichnet) gelingt mit herkömmlichen Rundläuferpressen, indem eine Transferund Zentriervorrichtung angebracht wird, die einen vorverpreßten Kern in die befüllte Matrize einlegt, bevor die gesamte Mischung endverpreßt wird.

Die Form der Matrizenbohrungen und der Stempeloberflächen kann innerhalb weiter Grenzen variiert werden. So sind runde, ovale und eckige Tabletten mit planer ober gewölbter Oberfläche, oder mit abgeschrägten Kanten herstellbar.

Aus der Batterieherstellung ist die Produktion von ringförmigen "Tabletten" mit der Rundläufertechnologie bekannt. Die später mit einem Kohlstift zu befüllenden Graphit-Mangan-Ringe werden dabei mit einem ringförmigen Unterstempel hergestellt, wobei in die Matrize von unten ein starr befestigter Mittendorn hineinragt, an dem sich der Unterstempel auf- und abwärts vorbeibewegt. Dabei ist der Unterstempel in seinem Unterteil mit zwei Kerben versehen, die an der Halterung des Mittendorns vorbeigleiten.

Zu den vorstehend genannten Herstellverfahren existiert umfangreiches Schrifftum, insbesondere auf dem Anwendungsgebiet der Pharmazie. Neben Lehrbüchern zur pharmazeutischen Technologie wie *"****Hagers Handbuch der pharmazeutischen Praxis*",** *Band 2, 5. Aufl. 1991, Springer Verlag Berlin, Heidelberg, New York, London, Paris, Tokyo, Hong Kong, Barcelona, Budapest, Seite 938ff.* oder *K. H. Bauer, K.-H. Frömming, C. Führer "****Pharmazeutische Technologie",*** *4. Auflage 1997*, *Gustav Fischer Verlag Stuttgart, Jena, Lübeck, Ulm, Seite 299ff.* existieren zahlreiche Spezialmonographien, beispielsweise das dreibändige Werk *H. A. Liebermann, L. Lachmann, J. B. Schwartz* ***"Pharmaceutical Dosage Forms - Tablets",*** *Verlag M. Dekker Inc., New York, 1989.*

Auch in der Patentliteratur existiert eine Vielzahl von Veröffentlichungen, die sich mit der Herstellung mehrschichtiger oder -phasiger Tabletten beschäftigen. So offenbart das US-Patent **5,158,728** (Sanderson et al) eine Vorrichtung zum Pressen zweisschichtiger Tabletten, die eine spezielle Form aufweisen. Dreischichtige Tabletten, die bei der Batterieherstellung Verwendung finden, werden in der **EP-A-0 307 209** (Sharp) beschrieben. Eine Rundläuferpresse für das Pressen zweischichtiger Tabletten wird auch in der deutschen Gebrauchsmusteranmeldung **G 92 08 040.5 U1** beschreiben. Diese Presse weist eine Zwischenentnahmestation mit einem verstellbaren Auswerferkurvensegment und einer Ablaufweiche auf, durch die die Preßlinge einem Schlechtkanal oder einer Prüfstation zugelenkt werden können.

Ein Verfahren für die Herstellung zweiphasiger Muldentabletten mittels einer Rundläuferpresse, welche Unterstempel mit zwei unabhängig voneinander und parallel zueinander beweglichen Teilstempeln aufweist, wird durch das US-Patent **3,840,631** offenbart.

Für die Herstellung von Manteltabletten oder Ringkerntabletten sind in der Regel mehrere Verfahrensschritte notwendig, wobei die Kern- bzw. Ringfüllungs-Segmente entweder separat durch Tablettierung hergestellt werden müssen oder im Falle von Muldentabletten durch spezielle Technologien auf die Tablette aufgebracht werden.

Der vorliegenden Erfindung lag die Aufgabe zugrunde, eine Vorrichtung und ein Verfahren bereitzustellen, die es ermöglichen, zwei- und mehrphasige Tabletten auf einer üblichen Rundläuferpresse herzustellen, ohne daß aufwendige Transfer- und Zentriervorrichtungen oder die Vorverpressung einzusetzender Kerne benötigt werden. Insbesondere sollte die Herstellung optisch ansprechender Ringkerntabletten, Manteltabletten oder Muldentabletten (Punkttabletten) auf eine einfache und kostengünstige Weise und in großen Stückzahlen möglich gemacht werden.

Gegenstand der vorliegenden Erfindung ist eine Rundläuferpresse zum Pressen mehrphasiger Tabletten mit einem Stempelsatz aus Ober- und Unterstempel, dadurch gekennzeichnet, daß sowohl der Oberstempel aus auch der Unterstempel aus zwei unabhängig voneinander und parallel zueinander beweglichen Teilstempeln aufgebaut ist.

Bei dieser erfindungsgemäßen Vorrichtung benötigt jeder Teilstempel des Unterstempels und Oberstempels selbstverständlich eine eigene Stempelbahn, damit eine voneinander unabhängige Beweglichkeit der Teilstempel gewährleistet ist. Auch die übrigen Teile, die bei herkömmlichen Rundläuferpressen für die Bewegung der einteiligen Unterstempel und Oberstempel sorgen, wie beispielsweise Druckrollen und optional einzusetzende Niederzugschienen sind im Rahmen der vorliegenden Erfindung in jeweils zwei Ausführungen erforderlich.

Die Aufteilung des Unterstempels und Oberstempels in zwei unabhängig voneinander wirkende Teilstempel kann auf unterschiedliche Art und Weise erfolgen. So ist es beispielsweise denkbar, einen eckigen oder runden Stempel im ersten Fall durch zwei halbe Ecken oder zwei Dreiecke und im letzten Fall durch zwei Halbkreise zu ersetzen. Dies führt zu mehrphasigen Tabletten, bei denen mindestens eine Stirnseite eine Trennungslinie aufweist, die durch die Verwendung unterschiedlich eingefärbter Vorgemische optisch hervorgehoben werden kann. Der Vielfalt der Ausgestaltungsmöglichkeiten sind hierbei kaum Grenzen gesetzt, so daß das obengenannte Beispiel dahingehend erweitert werden kann, daß die Trennungslinie keine Gerade ist, sondern eine mehr oder minder stark und häufig gekrümmte Kurve. Auf diese Weise sind die unterschiedlichsten Formkörper herstellbar.

Da mit zunehmender Komplexität der Preßflächen der beiden Teilstempel anwendungstechnische Probleme wie Stempelanbackungen, Deckeln, übermäßiger Verschleiß usw. stark ansteigen, ist eine erfindungsgemäße Vorrichtung bevorzugt, bei der der Ober- und Unterstempel aus einen Mittendorn und einem diesen Mittendorn umschließenden Ringstempel besteht.

Auch hierbei gibt es eine Vielzahl von Gestaltungsmöglichkeiten für Ringstempel und Mittendorn. So kann bei einer eckigen Matrize und einem auf diese Matrize angepaßten Ringstempel mit eckigem Grundriß der Stirnfläche (Preßfläche) ein eckiger oder ein runder Mittendorn verwendet werden, was zu unterschiedlichen Aufsichten auf die Oberfläche der hergestellten Tabletten führt. Analog sind runde oder ovale Tabletten mit einem eckigen oder runden Einsatz herstellbar. Aus den obengenannten Gründen ist eine erfindungsgemäße Vorrichtung bevorzugt, bei der der Mittendorn eine ellipsenförmige, kreisrunde oder rechteckige Stirnfläche aufweist.

Bei dieser Ausgestaltungsform weist der den Mittendorn umgebende Ringstempel eine ebenfalls kreisrunde Bohrung auf, in der der Mittendom frei auf und ab bewegt werden kann. Diese Geometrie hat keinen Einfluß auf den äußeren Grundriß der Stirnfläche der Matrize (und damit des Ringstempels) - hier sind nach wie vor sämtliche Formvarianten möglich. Aus ästhetischen Gründen sind allerdings ebenfalls kreisrunde Matrizen zu bevorzugen. Bei dieser speziellen Ausgestaltungsform weist der Ringstempel einen Grundriß in Form zweier konzentrischer Kreise auf.

Die nachfolgenden Ausführungen beziehen sich nur noch auf die bevorzugte Ausführungsform mit Ringstempel und Mittendorn, lassen sich aber völlig analog auf andere Teilstempelgeometrien übertragen. Hierbei wären Begriffe wie "Ring" und "Kern" ersetzbar durch "linker Bereich" und "rechter Bereich" oder Analoga, die dem Fachmann geläufig sind.

Bei der Verpressung wird üblicherweise zuerst der Ringstempel abgesenkt und ein Ring aus zu tablettierendem Material vorgeformt. Die umgekehrte Verfahrensweise, zuerst den Mittendorn abzusenken, ist prinzipiell auch möglich, doch ist das nachfolgende Befüllen eines ringförmigen Bereichs um den entstandenen "Kern" technisch aufwendiger als das Befüllen einer verengten "Matrize" innerhalb eines vorverpreßten Rings.

Das Verpressen des zuerst in die Matrize eingefüllten Vorgemischs kann mit einem einzigen Oberstempel erfolgen, der beim Vorpressen des Rings den Mittendorn nach unten drückt. Bevorzugt ist erfindungsgemäß aber eine Vorrichtung, bei der der Oberstempel analog zum Unterstempel aufgebaut ist. Durch diese Ausführungsform kann das bei herkömmlichen Tablettenpressen bewährte Zusammenspiel von Ober- und Unterstempel beim Preßvorgang auch erfindungsgemäß genutzt werden. Durch den beim Vorpressen des Rings feststehenden Mittendorn wird die Stabilität des Rings verbessert.

Ein weiterer Gegenstand der vorliegenden Erfindung ist ein Verfahren zur Herstellung mehrphasiger Formkörper, dadurch gekennzeichnet, daß sich während des Preßvorgangs die Teilstempel der Ober- und/oder Unterstempel unabhängig voneinander und parallel zueinander bewegen.

Auch beim erfindungsgemäßen Verfahren ist die bereits oben als bevorzugt hervorgehobene Geometrie des Unterstempels der Gegenstand einer bevorzugten Ausführungsform des erfindungsgemäßen Verfahrens, bei dem der Unterstempel aus einen Mittendorn und einem diesen Mittendorn umschließenden Ringstempel besteht. Völlig analog zur erfindungsgemäßen Vorrichtung ist es auch beim erfindungsgemäßen Verfahren bevorzugt, daß der Oberstempel analog zum Unterstempel aufgebaut ist, also ebenfalls aus zwei unabhängig voneinander und parallel zueinander beweglichen Teilen, vorzugsweise einem Mittendorn und einem diesen Mittendorn umschließenden Ringstempel, besteht.

Das erfindungsgemäße Verfahren eignet sich insbesondere zur Herstellung von Tabletten, bei denen es erwünscht ist, bestimmte Inhaltsstoffe in unterschiedlichen Bereichen der Tablette unterzubringen. Neben der Herstellung pharmazeutischer Zubereitungsformen eignet sich das erfindungsgemäße Verfahren insbesondere zur Herstellung von Wasch- und Reinigungsmittelformkörpern, wie beispielsweise Waschmitteltabletten, Reinigungsmitteltabletten für das maschinelle Geschirrspülen, Bleichmitteltabletten, Fleckensalztabletten usw..

Je nachdem, wieviele unterschiedliche Phasen die mit dem erfindungsgemäßen Verfahren hergestellten Tabletten aufweisen sollen, müssen unterschiedlich viele Füllstationen für die Vorgemische der einzelnen Phasen an der Rundläuferpresse installiert werden. Diese n Füllschuhe werden vorzugsweise im Abstand von 360/n Grad an der Rundläuferpresse angeordnet. Besonders bevorzugte Ausführungsformen des erfindungsgemäßen Verfahrens verwenden zwei Füllschuhe im Abstand von 180° oder drei Füllschuhe im Abstand von jeweils 120°. Die erstgenannten Verfahren dienen insbesondere zur Herstellung von optional mehrschichtigen Ringkerntabletten, in denen die oberste Schicht und der Kern identisch zusammengesetzt sind, während mit der zweiten Verfahrensvariante mehrschichtige Ringkemtabletten herstellbar sind, die eine unterschiedliche Zusammensetzung auch in den einzelnen Schichten aufweisen.

Erfindungsgemäß bevorzugt ist also beispielsweise ein Verfahren, bei dem die Rundläuferpresse zwei Füllschuhe aufweist, wobei beim ersten Füllschuh der Ringstempel 2 abgesenkt ist und der Mittendorn 3 bündig mit der Matrize 1 abschließt, so daß das Vorgemisch 4 für die erste Phase in eine ringförmige Matrize eingefüllt und vorverdichtet wird, und beim zweiten Füllschuh der Mittendorn 3 auf das Niveau des Ringstempels 2 abgesenkt wird, so daß Vorgemisch für die zweite Phase 6 in die Mitte eines vorverpreßten, bündig mit der Matrize 1 abschließenden Rings 5 eingefüllt wird und eine Endverpressung zu einer Ringkerntablette 7 erfolgt.

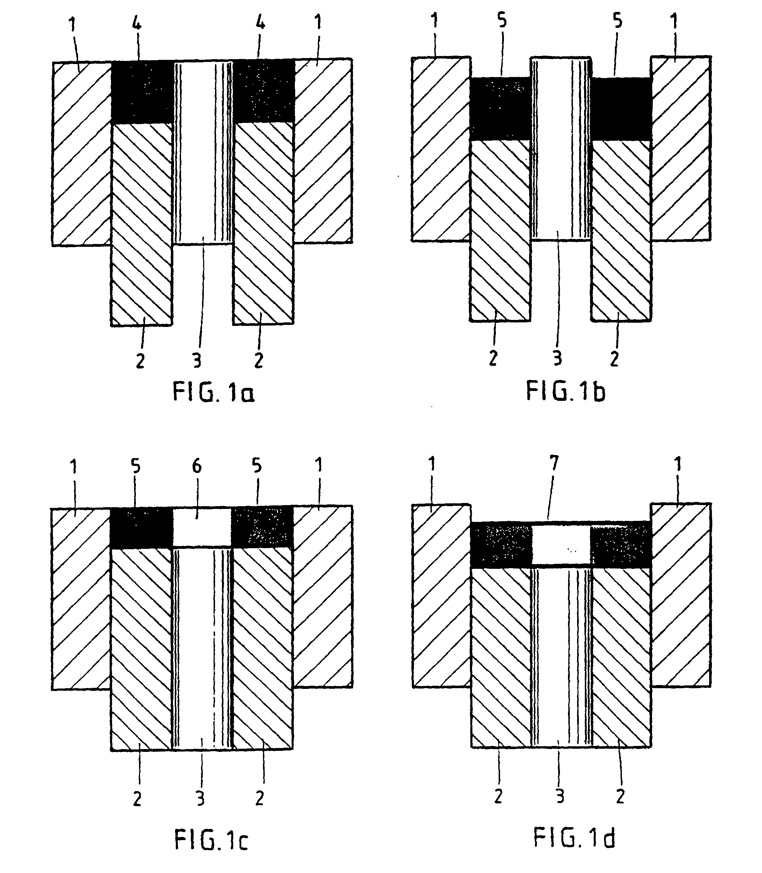

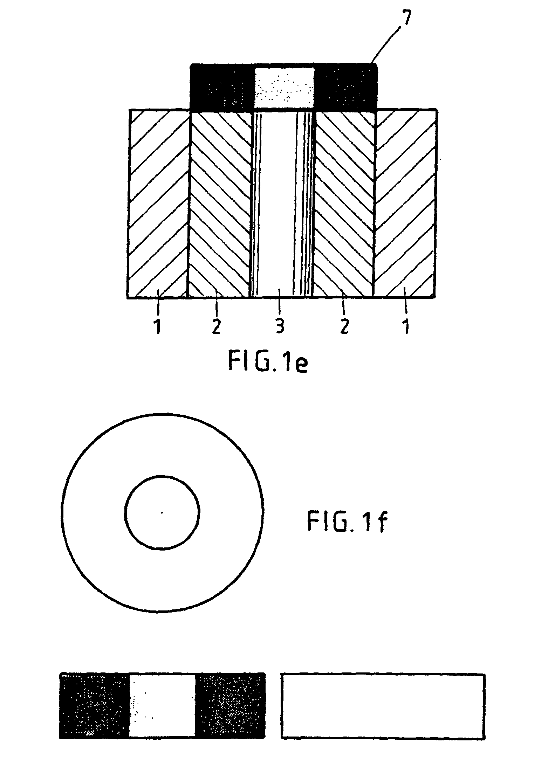

Dieser Verfahrensablauf ist in der Abbildung 1 verdeutlicht, die jeweils vertikale Schnitte durch eine einzelne Matrize 1 während eines vollständigen Umlaufs zeigt. In der Startstellung (Abbildung 1 a, 0°) ist der Mittendorn 3 bis an die Oberkante der Matrize 1 (nachfolgend "Matrizenoberfläche" genannt) ausgefahren und der Ringstempel 2 abgesenkt. In die auf diese Weise gebildete ringförmige Vertiefung wird das Vorgemisch 4 für die erste Phase eingefüllt und bei Erreichen der ersten Druckrolle durch den vorzugsweise ebenfalls ringförmig ausgebildeten Oberstempel vorverdichtet (Abbildung 1 b). Bei Erreichen des zweiten Füllschuhs (Abbildung 1 c, 180°) ist der Mittendorn 3 auf das Niveau des Ringstempels 2 abgesenkt, und das Vorgemisch 6 für die zweite Phase kann in die aus der ersten Phase gebildete Vertiefung der vorverpreßten Ringtablette 5 eingefüllt werden. Die zweite Druckrolle verpreßt unter Absenken beider Oberstempelteile die beiden Vorgemische zu einer Ringekerntablette 7 (Abbildung 1 d), die nachfolgend ausgestoßen (Abbildung 1 e) und abtransportiert wird. Abbildung 1 f zeigt schematisch die Aufsicht, ein Schnittbild und die Seitenansicht einer solcherart hergestellten Tablette.

Die bevorzugte erfindungsgemäße Verfahrensvariante, bei der drei Füllschuhe eingesetzt werden, ist dadurch gekennzeichnet, daß die Rundläuferpresse drei Füllschuhe aufweist, wobei beim ersten Füllschuh der Ringstempel 2 abgesenkt ist und der Mittendorn 3 bündig mit der Matrize 1 abschließt, so daß das Vorgemisch 4 für die erste Phase in eine ringförmige Matrize eingefüllt und vorverdichtet wird, beim zweiten Füllschuh der Mittendorn 3 auf das Niveau des Ringstempels 2 abgesenkt wird, so daß Vorgemisch 6 für die zweite Phase in die Mitte des vorverpreßten, bündig mit der Matrize abschließenden Rings 5 eingefüllt und vorverdichtet wird, beim dritten Füllschuh Ringstempel 2 und Mittendorn 3 weiter abgesenkt werden, so daß das Vorgemisch 8 für die dritte Phase als Deckschicht über die vorverpreßte und abgesenkte Ringkerntablette 7 eingefüllt wird und eine Endverpressung zu einer dreiphasigen Tablette 9 erfolgt.

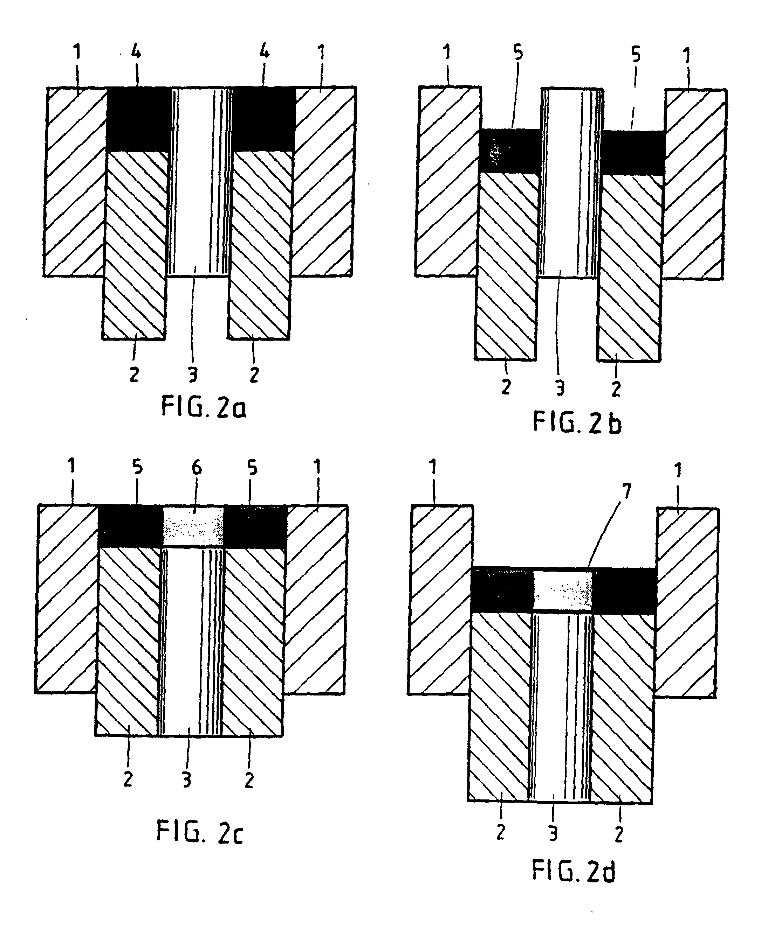

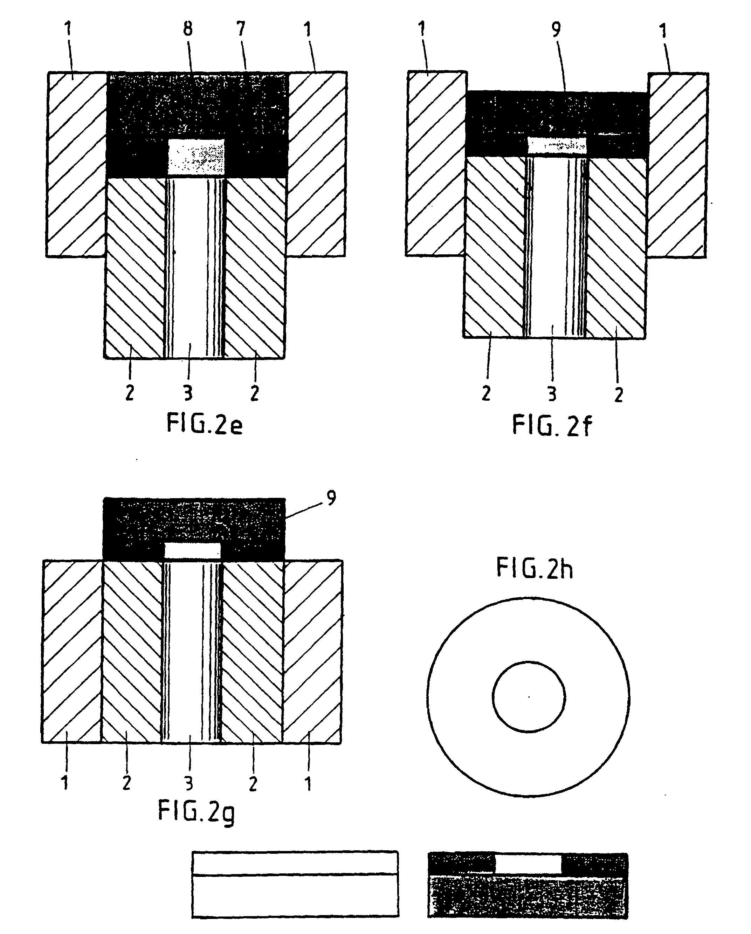

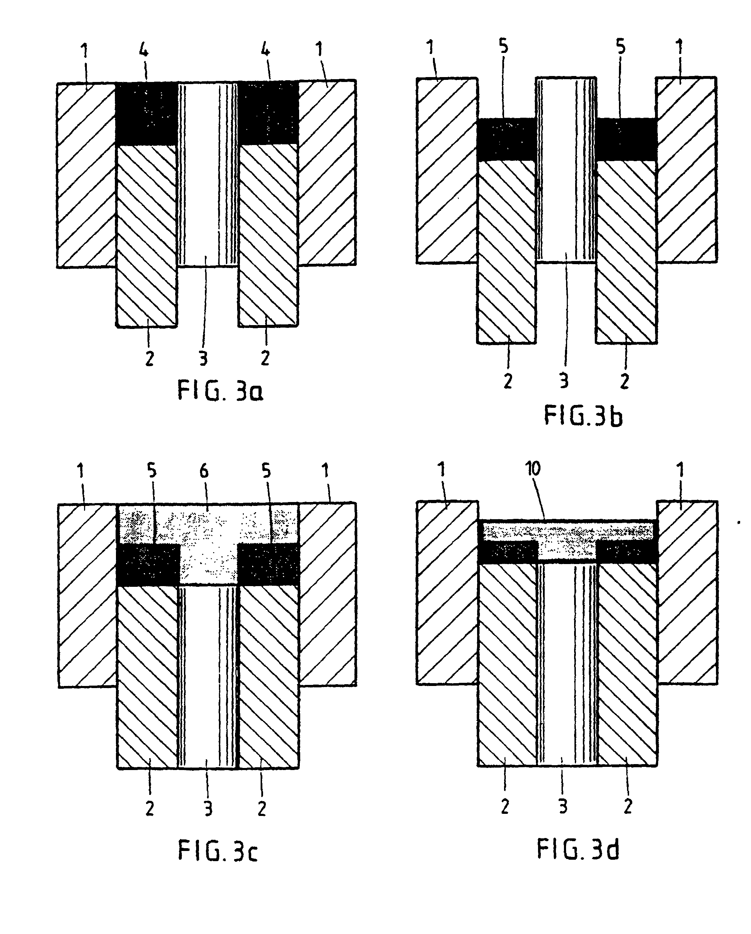

Dieser Verfahrensablauf ist in der Abbildung 2 verdeutlicht, die jeweils vertikale Schnitte durch eine einzelne Matrize 1 während eines vollständigen Umlaufs zeigt. In der Startstellung (Abbildung 2 a, 0°) ist der Mittendorn 3 bis an die Oberkante der Matrize 1 ausgefahren und der Ringstempel 2 abgesenkt. In die auf diese Weise gebildete ringförmige Vertiefung wird das Vorgemisch 4 für die erste Phase eingefüllt und bei Erreichen der ersten Druckrolle durch den vorzugsweise ebenfalls ringförmig ausgebildeten Oberstempel vorverdichtet (Abbildung 1 b). Bei Erreichen des zweiten Füllschuhs (Abbildung 2 c, 120°) ist der Mittendorn 3 auf das Niveau des Ringstempels 2 abgesenkt, und das Vorgemisch 6 fiir die zweite Phase kann in die aus der ersten Phase gebildete Vertiefung eingefüllt werden. Die zweite Druckrolle verpreßt unter Absenken beider Oberstempelteile die beiden Vorgemische zu einer Ringekerntablette 7 (Abbildung 2 d). In die nach Abheben des Oberstempels und/oder Absenken der Unterstempelteile gebildete neue Vertiefung wird nachfolgend aus dem dritten Füllschuh (Abbildung 2 e, 240°) das Vorgemisch 8 für die dritte Phase eingefüllt, bei Erreichen der dritten Druckrolle (Abbildung 2 f) unter Absenken beider Oberstempelteile verpreßt und nachfolgend in Form einer Dreiphasentablette 9 ausgestoßen (Abbildung 2 g) und abtransportiert. Abbildung 2 h zeigt schematisch die Aufsicht, ein Schnittbild und die Seitenansicht einer solcherart hergestellten Tablette.

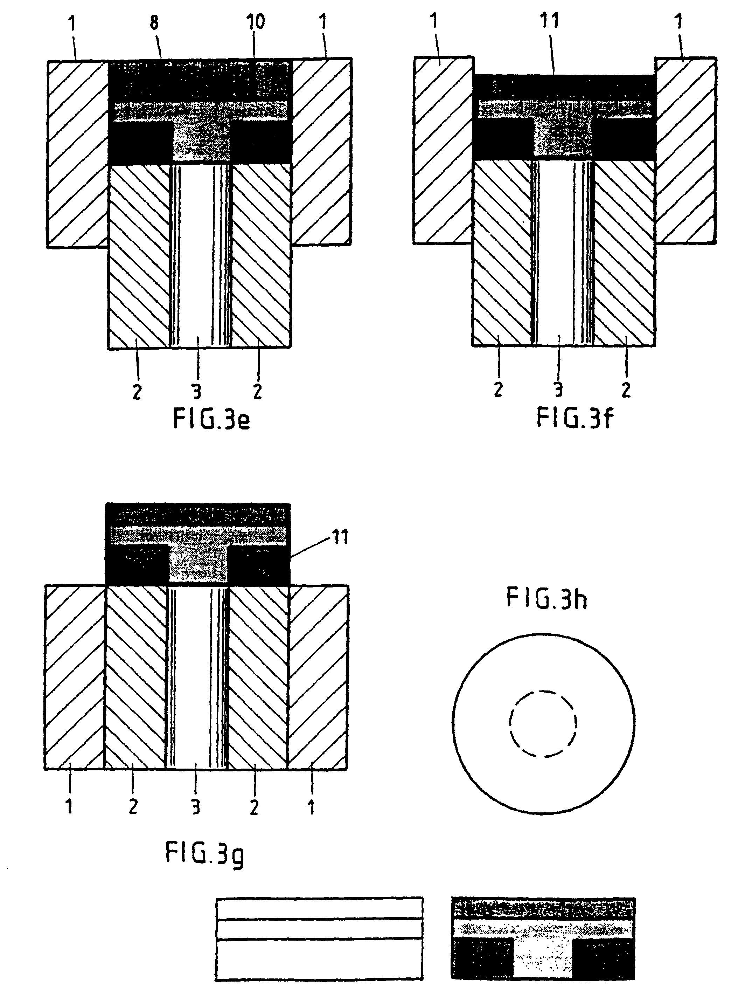

Wird das Vorgemisch für die zweite Phase in dem in der Abbildung 1 skizzierten Verfahren über das Niveau des vorverpreßten Rings hinaus eingefüllt oder werden bei dem in Abbildung 2 skizzierten Verfahren für die zweite und dritte Phase identische Vorgemische eingesetzt, so gelangt man zu pseudo-Dreiphasentabletten, bei denen die untere Schicht und der Kern aus dem gleichen Material bestehen ("Zweiphasen-T-Tabletten"). Die geschilderte Abwandlung des in der Abbildung 1 skizzierten Verfahrens läßt sich dazu nutzen, pseudo-Vierphasentabletten herzustellen, indem man die zwei Füllschuhe im Abstand von 120° anordnet, wie beschrieben verfährt und einen dritten Füllschuh einbaut, der eine neue Schicht auf die pseudo-Dreiphasentablette aufbringt. Diese Verfahrensvariante unter Verwendung von drei Füllschuhen ist in der Abbildung 3 skizziert und bedarf keiner weiteren Erklärung. Weitere erfindungsgemäß bevorzugte Verfahren sind daher die Herstellverfahren für pseudo-Dreiphasentabletten unter Einsatz von zwei Füllschuhen und Herstellverfahren für pseudo-Vierphasentabletten unter Einsatz von drei Füllschuhen. Das pseudo-Dreiphasentabletten-Herstellverfahren ist dabei dadurch gekennzeichnet, daß die Rundläuferpresse zwei Füllschuhe aufweist, wobei beim ersten Füllschuh der Ringstempel 2 abgesenkt ist und der Mittendorn 3 bündig mit der Matrize 1 abschließt, so daß das Vorgemisch 4 für die erste Phase in eine ringförmige Matrize eingefüllt und vorverdichtet wird, und beim zweiten Füllschuh der Mittendorn 3 auf das Niveau des Ringstempels 2 abgesenkt wird und Ringstempel 2 und Mittendorn 3 weiter abgesenkt werden, so daß das Vorgemisch 6 für die zweite Phase sowohl als Ringfüllung als auch als Deckschicht auf die vorverpreßte und unter die Matrizenoberfläche abgesenkte Ringtablette 5 eingefüllt wird und eine Endverpressung zu einer zweiphasigen Tablette 10 erfolgt. Diese Verfahrensweise läßt sich aus Abbildung 3 entnehmen, wenn der bei der zweiten Druckrolle (Abbildung 3d) gebildete Formkörper nicht vorverdichtet und weiterverarbeitet, sondern endverpreßt und ausgestoßen wird.

Die Herstellung pseudo-vierphasiger Tabletten gelingt durch ein Verfahren, bei dem die Rundläuferpresse drei Füllschuhe aufweist, wobei beim ersten Füllschuh der Ringstempel 2 abgesenkt ist und der Mittendorn 3 bündig mit der Matrize 1 abschließt, so daß das Vorgemisch 4 für die erste Phase in eine ringförmige Matrize eingefüllt und vorverdichtet wird, und beim zweiten Füllschuh der Mittendorn 3 auf das Niveau des Ringstempels 2 abgesenkt wird und Ringstempel 2 und Mittendorn 3 weiter abgesenkt werden, so daß das Vorgemisch 6 für die zweite Phase sowohl als Ringfüllung als auch als Deckschicht auf die vorverpreßte und unter die Matrizenoberfläche abgesenkte Ringtablette 5 eingefüllt und vorverdichtet wird, beim dritten Füllschuh Ringstempel 2 und Mittendorn 3 weiter abgesenkt werden, so daß das Vorgemisch 8 für die dritte Phase als Deckschicht über die vorverpreßte und unter die Matrizenoberfläche abgesenkte Zweischicht-Ringkerntablette 10 eingefüllt wird und eine Endverpressung zu einer pseudo-vierphasigen Tablette 11 erfolgt.

### Bezugszeichenliste

- 1: Matrize
- 2: Ringstempel
- 3: Mittendorn
- 4: Vorgemisch für die erste Phase
- 5: Ringtablette, vorverpreßt
- 6: Vorgemisch für die zweite Phase
- 7: Ringkerntablette
- 8: Vorgemisch für die dritte Phase
- 9: dreiphasige Tablette
- 10: "Zweiphasen-T-Tablette", vorverpreßt
- 11: pseudo-Vierphasentablette

## Patentansprüche

1. Rundläuferpresse zum Pressen mehrphasiger Tabletten mit einem Stempelsatz aus Oberund Unterstempeln, **dadurch gekennzeichnet, daß** sowohl der Oberstempel als auch der Unterstempel aus zwei unabhängig voneinander und parallel zueinander beweglichen Teilstempeln aufgebaut ist.

2. Vorrichtung nach Anspruch 1, **dadurch gekennzeichnet, daß** Ober- und Unterstempel aus einem Mittendorn und einem diesen Mittendorn umschließenden Ringstempel bestehen.

3. Verfahren zur Herstellung mehrphasiger Formkörper auf einer Vorrichtung nach einem der Ansprüche 1 oder 2, **dadurch gekennzeichnet, daß** sich während des Preßvorgangs die Teilstempel der Ober- und/oder Unterstempel unabhängig voneinander und parallel zueinander bewegen.

4. Verfahren nach Anspruch 3, **dadurch gekennzeichnet, daß** die Rundläuferpresse zwei Füllschuhe aufweist, wobei beim ersten Füllschuh der Ringstempel (2) abgesenkt ist und der Mittendorn (3) bündig mit der Matrize (1) abschließt, so daß das Vorgemisch (4) für die erste Phase in eine ringförmige Matrize eingefüllt und vorverdichtet wird, und beim zweiten Füllschuh der Mittendorn (3) auf das Niveau des Ringstempels (2) abgesenkt wird, so daß Vorgemisch (6) für die zweite Phase in die Mitte eines vorverpreßten, bündig mit der Matrize (1) abschließenden Rings (5) eingefüllt wird und eine Endverpressung zu einer Ringkerntablette (7) erfolgt.

5. Verfahren nach Anspruch 3, **dadurch gekennzeichnet, daß** die Rundläuferpresse drei Füllschuhe aufweist, wobei beim ersten Füllschuh der Ringstempel (2) abgesenkt ist und der Mittendorn (3) bündig mit der Matrize (1) abschließt, so daß das Vorgemisch (4) für die erste Phase in eine ringförmige Matrize eingefüllt und vorverdichtet wird, beim zweiten Füllschuh der Mittendorn (3) auf das Niveau des Ringstempels (2) abgesenkt wird, so daß Vorgemisch (6) für die zweite Phase in die Mitte des vorverpreßten, bündig mit der Matrize abschließenden Rings (5) eingefüllt und vorverdichtet wird, beim dritten Füllschuh Ringstempel (2) und Mittendorn (3) weiter abgesenkt werden, so daß das Vorgemisch (8) für die dritte Phase als Deckschicht über die vorverpreßte und abgesenkte Ringkerntablette (7) eingefüllt wird und eine Endverpressung zu einer dreiphasigen Tablette (9) erfolgt.

6. Verfahren nach Anspruch 3, **dadurch gekennzeichnet, daß** die Rundläuferpresse zwei Füllschuhe aufweist, wobei beim ersten Füllschuh der Ringstempel (2) abgesenkt ist und der Mittendorn (3) bündig mit der Matrize (1) abschließt, so daß das Vorgemisch (4) für die erste Phase in eine ringförmige Matrize eingefüllt und vorverdichtet wird, und beim zweiten Füllschuh der Mittendorn (3) auf das Niveau des Ringstempels (2) abgesenkt wird und Ringstempel (2) und Mittendorn (3) weiter abgesenkt werden, so daß das Vorgemisch (6) für die zweite Phase sowohl als Ringfüllung als auch als Deckschicht auf die vorverpreßte und unter die Matrizenoberfläche abgesenkte Ringtablette (5) eingefüllt wird und eine Endverpressung zu einer zweiphasigen Tablette (10) erfolgt.

7. Verfahren nach Anspruch 3, **dadurch gekennzeichnet, daß** die Rundläuferpresse drei Füllschuhe aufweist, wobei beim ersten Füllschuh der Ringstempel (2) abgesenkt ist und der Mittendorn (3) bündig mit der Matrize (1) abschließt, so daß das Vorgemisch (4) für die erste Phase in eine ringförmige Matrize eingefüllt und vorverdichtet wird, und beim zweiten Füllschuh der Mittendorn (3) auf das Niveau des Ringstempels (2) abgesenkt wird und Ringstempel (2) und Mittendorn (3) weiter abgesenkt werden, so daß das Vorgemisch (6) für die zweite Phase sowohl als Ringfüllung als auch als Deckschicht auf die vorverpreßte und unter die Matrizenoberfläche abgesenkte Ringtablette (5) eingefüllt und vorverdichtet wird, beim dritten Füllschuh Ringstempel (2) und Mittendorn (3) weiter abgesenkt werden, so daß das Vorgemisch (8) für die dritte Phase als Deckschicht über die vorverpreßte und unter die Matrizenoberfläche abgesenkte Zweischicht-Ringkerntablette (10) eingefüllt wird und eine Endverpressung zu einer pseudo-vierphasigen Tablette (11) erfolgt.

## Claims

1. A rotary press for pressing multiphase tablets comprising a punch set of top and bottom punches, **characterized in that** both the top punch and the bottom punch are made up of two part punches designed to move independently of and parallel to one another.

2. A press as claimed in claim 1, **characterized in that** the top and bottom punches consist of a centre mandrel and an annular punch surrounding the centre mandrel.

3. A process for the production of multiphase tablets using the press claimed in claim 1 or 2, **characterized in that** the part punches of the top and/or bottom punches move independently of and parallel to one another during pressing.

4. A process as claimed in claim 3, **characterized in that** the rotary press comprises two filling shoes, the annular punch (2) being lowered in the case of the first filling shoe and the centre mandrel (3) ending flush with the cavity block (1), so that the premix (4) for the first phase is introduced into an annular cavity and precompressed, and the centre mandrel (3) being lowered to the level of the annular punch (2) in the case of the second filling shoe, so that the premix (6) for the second phase is introduced into the middle of a prepressed ring (5) ending flush with the cavity block (1) and the whole is pressed to form a ring/core tablet (7).

5. A process as claimed in claim 3, **characterized in that** the rotary press comprises three filling shoes, the annular punch (2) being lowered in the case of the first filling shoe and the centre mandrel (3) ending flush with the cavity block (1), so that the premix (4) for the first phase is introduced into an annular cavity and precompressed; the centre mandrel (3) being lowered to the level of the annular punch (2) in the case of the second filling shoe, so that the premix (6) for the second phase is introduced into the middle of a prepressed ring (5) ending flush with the cavity block (1) and precompressed; and the annular punch (2) and centre mandrel (3) being further lowered in the case of the third filling shoe, so that the premix (8) for the third phase is introduced as a cover layer over the precompressed and lowered ring/core tablet (7) and the whole is pressed to form a three-phase tablet (9).

6. A process as claimed in claim 3, **characterized in that** the rotary press comprises two filling shoes, the annular punch (2) being lowered in the case of the first filling shoe and the centre mandrel (3) ending flush with the cavity block (1), so that the premix (4) for the first phase is introduced into an annular cavity and precompressed and the centre mandrel (3) being lowered to the level of the annular punch (2) in the case of the second filling shoe and the annular punch (2) and centre mandrel (3) being further lowered in the case of the second filling shoe, so that the premix (6) for the second phase is introduced both as an annular filling and as a cover layer onto the precompressed ring tablet (5) lowered beneath the surface of the cavity and the whole is pressed to form a two-phase tablet (10).

7. A process as claimed in claim 3, **characterized in that** the rotary press comprises three filling shoes, the annular punch (2) being lowered in the case of the first filling shoe and the centre mandrel (3) ending flush with the cavity block (1), so that the premix (4) for the first phase is introduced into an annular cavity and precompressed; the centre mandrel (3) being lowered to the level of the annular punch (2) in the case of the second filling shoe and the annular punch (2) and centre mandrel (3) being further lowered, so that the premix (6) for the second phase is introduced both as an annular filling and as a cover layer onto the precompressed ring tablet (5) lowered beneath the surface of the cavity and precompressed; and the annular punch (2) and centre mandrel (3) being further lowered in the case of the third filling shoe, so that the premix (8) for the third phase is introduced as a cover layer over the precompressed two-layer ring/core tablet (10) lowered beneath the surface of the cavity and the whole is pressed to form a pseudo-four-phase tablet (11).

## Revendications

1. Presse à table tournante pour le pressage de pastilles à plusieurs phases avec un jeu de pistons constitué d'un piston supérieur et un piston inférieur, **caractérisée en ce que** tant le piston supérieur qu'également le piston inférieur sont construits à partir de deux pistons partiels indépendants l'un de l'autre et mobiles parallèlement l'un à l'autre.

2. Dispositif suivant la revendication 1, **caractérisé en ce que** le piston supérieur et le piston inférieur sont constitués d'un poinçon central et d'un piston annulaire entourant ce poinçon central.

3. Procédé de fabrication de corps moulés à plusieurs phases sur un dispositif suivant l'une des revendications 1 ou 2, **caractérisé en ce que**, pendant l'opération de pressage, les pistons partiels des pistons supérieurs et/ou des pistons inférieurs se déplacent indépendamment l'un de l'autre et parallèlement l'un à l'autre.

4. Procédé suivant la revendication 3, **caractérisé en ce que** la presse à table tournante présente deux sabots de remplissage, le piston annulaire (2) étant abaissé et le poinçon central (3) se terminant à fleur avec la matrice 1 pour le premier sabot de de sorte que le mélange préalable (4) pour la première phase est introduit dans une matrice annulaire et précompacté, et le poinçon central (3) étant abaissé pour le deuxième sabot de remplissage au niveau du piston annulaire (2), de sorte que le mélange préalable (6) pour la deuxième phase est introduit au centre d'un anneau précompacté (5) se terminant à fleur avec la matrice (1) et qu'un pressage final a lieu pour donner une pastille à noyau et anneau (7).

5. Procédé suivant la revendication 3, **caractérisé en ce que** la presse à table tournante présente trois sabots de remplissage, le piston annulaire (2) étant abaissé et le poinçon central (3) se terminant à fleur avec la matrice (1) pour le premier sabot de remplissage, de sorte que le mélange préalable (4) pour la première phase est introduit dans une matrice annulaire et précompacté, le poinçon central (3) étant abaissé au niveau du piston annulaire (2) pour le deuxième sabot de remplissage, de sorte que le mélange préalable (6) pour la deuxième phase est introduit au centre de l'anneau précompacté (5) se terminant à fleur avec la matrice (1) et précompacté, le piston annulaire (2) et le poinçon central (3) étant abaissés plus fortement pour le troisième sabot de remplissage, de sorte que le mélange préalable (8) pour la troisième phase est introduit comme couche de couverture sur la pastille à noyau et anneau (7) précompactée et qu'un pressage final suit pour donner une pastille à trois phases (9).

6. Procédé suivant la revendication 3, **caractérisé en ce que** la presse à table tournante présente deux sabots de remplissage, le piston annulaire (2) étant abaissé et le poinçon central (3) se terminant à fleur avec la matrice (1) pour le premier sabot de remplissage, de sorte que le mélange préalable (4) pour la première phase est introduit dans une matrice annulaire et précompacté, et le poinçon central (3) étant abaissé au niveau du piston annulaire (2) et le piston annulaire (2) et le poinçon central (3) étant abaissés plus fortement pour le deuxième sabot, de remplissage, de sorte que le mélange préalable (6) pour la deuxième phase est introduit tant comme remplissage annulaire que comme couche de couverture sur la pastille annulaire (5) précompactée et abaissée en dessous de la surface de la matrice et qu'un pressage final a lieu pour donner une pastille à deux phases (10).

7. Procédé suivant la revendication 3, **caractérisé en ce que** la presse à table tournante présente trois sabots de remplissage, le piston annulaire (2) étant abaissé et le poinçon central (3) se terminant à fleur avec la matrice (1) pour le premier sabot de remplissage, de sorte que le mélange préalable (4) pour la première phase est introduit dans une matrice annulaire et précompacté, le poinçon central (3) étant abaissé au niveau du piston annulaire (2) et le piston annulaire (2) et le poinçon central 3 étant abaissés plus fortement pour le deuxième sabot de remplissage, de sorte que le mélange préalable (6) pour la deuxième phase est introduit tant comme remplissage annulaire que comme couche de couverture sur la pastille annulaire (5) précompactée et abaissée en dessous de la surface de la matrice et est précompacté, le piston annulaire (2) et le poinçon central (3) étant abaissés plus fortement pour le troisième sabot de remplissage, de sorte que le mélange préalable (8) pour la troisième phase est introduit comme couche de couverture sur la pastille à deux couches à noyau et anneau (10) précompactée et abaissée en dessous de la surface de la matrice et qu'un pressage final a lieu pour donner une pseudo-pastille à quatre phases (11).
